(19) Europäisches Patentamt
European Patent Office
Office européen des brevets

(11) Numéro de publication: **0 173 634**
**B1**

# (12) FASCICULE DE BREVET EUROPÉEN

(45) Date de publication du fascicule du brevet: **31.08.88**

(51) Int. Cl.⁴: **C 07 D 295/08**, C 07 D 213/74, A 61 K 31/495

(21) Numéro de dépôt: **85450017.0**

(22) Date de dépôt: **23.07.85**

## (54) Nouveaux dérivés d'(arylpipérazinyléthylaminoéthoxy)-4 phénol, leur méthode de préparation et leur application thérapeutique.

(30) Priorité: **07.08.84 FR 8412580**
**04.07.85 FR 8510388**

(43) Date de publication de la demande:
**05.03.86 Bulletin 86/10**

(45) Mention de la délivrance du brevet:
**31.08.88 Bulletin 88/35**

(84) Etats contractants désignés:
**BE CH DE GB IT LI LU NL**

(56) Documents cité:
**DE-A-1 470 119**
**FR-M-2 446**

**CHEMICAL ABSTRACTS, vol. 57, no. 6, 17 septembre 1962, col. 7852g-7853a, Columbus, Ohio, US; L. BUCHEL et al.: "Relation between chemical constitution and pharmacological action. Influence of the nature of the amine on the pharmacological properties of several amino esters and several phenolic amino ethers"**

(73) Titulaire: **SOCIETE CORTIAL S.A., 7 rue de l'Armorique, F-75015 Paris (FR)**

(72) Inventeur: **Creuzet, Marie- Hélène, Résidence Jardin de Gambetta T 3, F-33000 Bordeaux (FR)**
Inventeur: **Feniou, Claude, 5 rue du Général Guillaumat, F-33600 Pessac (FR)**
Inventeur: **Guichard, Françoise, 26 avenue Eugène Delacroix, F-33700 Mérignac (FR)**
Inventeur: **Pontagnier, Henri, 21 rue Edouard Vaillant, F-33600 Pessac (FR)**
Inventeur: **Prat, Gisèle, Hameau de Noailles Villa 18, F-33400 Talence (FR)**

(74) Mandataire: **Tajan, Marie- Thérèse, LABORATOIRES SARGET Avenue du Président JF Kennedy, F-33701 Mérignac (FR)**

**EP 0 173 634 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne des dérivés phénoxypipéraziniques, leur méthode de préparation et leur application thérapeutique.

Les dérivés faisant l'objet de la présente invention sont représentés par la formule générale (I)

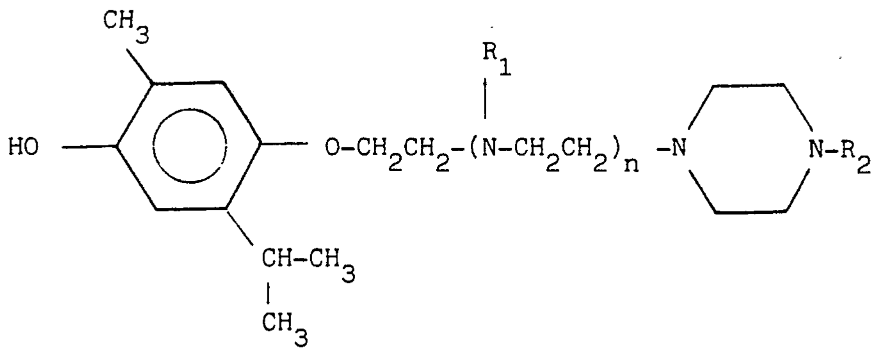

(I)

laquelle

n est égal à 0 ou 1,

$R_1$ représente un atome d'hydrogène ou un groupement méthyle,

$R_2$ représente soit un cycle phényle substitué ou non par un ou plusieurs substituants semblables ou différents choisis dans le groupe hydroxy, alcoxy de $C_1$ à $C_3$ (par exemple méthoxy, éthoxy, propoxy), trifluorométhyle, chloro, fluoro, ou alkyle de $C_1$ et $C_2$ (par exemple méthyle ou éthyle), soit un cycle pyridyl-2, -3 ou -4.

Ces produits peuvent être sous forme de base libre ou de sels d'acides pharmaceutiquement compatibles tels que leurs chlorhydrates.

On connait déjà des phénoxyalkylpipérazines; on peut citer par exemple la publication de R. Ratouis et coll. (J. Med. Chem., 1965, 271) et (Brevet FR-M-2446) décrivant des phénoxyalkylpipérazines à propriétés adrénolytiques, ou la publication de L. Cronenberger et coll., (Chimie Thérapeutique, 1966, 5-6, 289-291) décrivant la synthèse d'aryloxyalkylpipérazines sans toutefois décrire pour ces dérivés d'activité pharmacologique.

Nous avons maintenant découvert que les produits faisant l'objet de la présente invention substitués sur le noyau phénoxy par un méthyle en position 5, un isopropyle en position 2 et un hydroxy en position 4 présentent des propriétés pharmacologiques permettant leur utilisation en thérapeutique humaine et vétérinaire notamment en thérapeutique cardiovasculaire, psychotrope, hypolipidémiante et antiallergique. Le terme halogéno désignant, dans le texte qui suit, un atome de brome ou de chlore, les produits de formule I tels que n = 0 sont préparés de façon générale par une réaction mettant en jeu un (halogéno-2 éthoxy)-4 isopropyl-5 méthyl-2 phénol et une arylpipérazine de formule générale (II) dans laquelle

$R_2$ représente soit un cycle phényle substitué ou non par un ou plusieurs substituants semblables ou différents choisis dans le groupe hydroxy, alcoxy de $C_1$ à $C_3$ (par exemple méthoxy, éthoxy, propoxy), trifluorométhyle, chloro, fluoro, ou alkyle de $C_1$ et $C_2$ (par exemple méthyle ou éthyle), soit un cycle pyridyl-2, -3 ou -4.

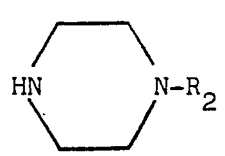

(II)

Les produits de formule générale (I) tels que n = 1 sont préparés par une réaction mettant en jeu une halogénoéthyl-1 aryl-4 pipérazine de formule générale (III)

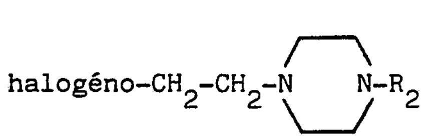

(III)

dans laquelle

$R_2$ représente soit un cycle phényle substitué ou non par un ou plusieurs substituants semblables ou différents choisis dans le groupe hydroxy, alcoxy de $C_1$ à $C_3$ (par exemple méthoxy, éthoxy, propoxy), trifluorométhyle, chloro, fluoro, ou alkyle de $C_1$ et $C_2$ (par exemple méthyle ou éthyle), soit un cycle pyridyl-2, -3 ou -4 et un isopropyl-5 méthyl-2 aminoéthoxy-4 phénol

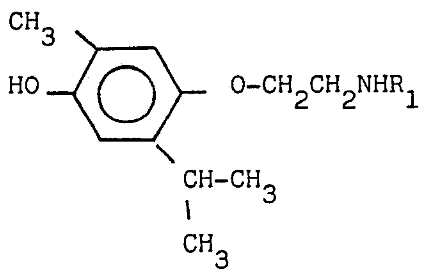

(IV)

de formule générale (IV) avec $R_1$ = H ou méthyle. Les produits de formule (I) tels que n = 1 peuvent également être préparés par une réaction mettant en jeu une aminoéthyl-1 aryl-4 pipérazine de formule générale (V)

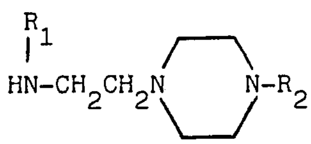

(V)

avec

$R_1$ = H ou méthyle et

$R_2$ représente soit un cycle phényle substitué ou non par un ou plusieurs substituants semblables ou différents choisis dans le groupe hydroxy, alcoxy de $C_1$ à $C_3$ (par exemple méthoxy, éthoxy, propoxy), trifluorométhyle, chloro, fluoro, ou alkyle de $C_1$ et $C_2$ ( par exemple méthyle ou éthyle), soit un cycle pyridyl-2, -3 ou -4, et un isopropyl-5 méthyl-2 halogénoéthoxy-4 phénol de formule générale (VI).

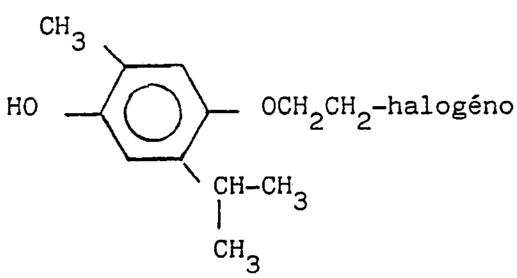

(VI)

Les exemples ci-après vont permettre de mieux illustrer la présente invention sans toutefois en restreindre la portée.

**Exemple 1**

Chlorhydrate de l'isopropyl-5 méthyl-2 ((méthoxy-2 phénylpipérazinyl)éthoxy)-4 phénol ou COR 2831; chlorhydrate du produit de formule (I) dans laquelle

n = 0,

$R_2$ = méthoxy-2 phényle

**Synthèse**

5,46 g de (bromo-2 éthoxy)-4 isopropyl-5 méthyl-2 phénol et 9,6 g de (méthoxy-2 phényl)pipérazine sont dissous dans 100 cm$^3$ d'éthanol. Cette solution est chauffée pendant 12 heures au reflux sous agitation. Les solvant est évaporé. Le résidu est repris dans une solution chlorhydrique aqueuse. Le dérivé bromé qui n'a pas réagi est extrait à l'aide de chloroforme. La solution aqueuse est neutralisée par de la soude et extraite par le chloroforme. La solution chloroformique est séchée sur $Na_2SO_4$; le solvant est évaporé et le produit obtenu est purifié en HPLC. Rendement 78 %. La base est transformée en chlorhydrate par l'action d'acide chlorhydrique gazeux en solution dans le chloroforme.

**Propriétés physicochimiques**

Point de fusion du chlorhydrate mesuré à l'appareil Mettler : 208°C. Spectre de RMN du chlorhydrate dans le $DMSOD_6$ : 1,2 ppm, 6 protons, doublet, $C(CH_3)_2$; 2,1 ppm, 3 protons, singulet, $CH_3$ sur le cycle phényle; 2,9 - 4,0 ppm, 14 protons, massif complexe, 5 $CH_2$-N + -CH- + $OCH_3$ à 3,8 ppm; 4,4 ppm, 2 protons, triplet mal résolu, $OCH_2$; 6,6 - 7,1 ppm, 6 protons, massif complexe, protons aromatiques; 5 - 10 ppm, 1 proton, pic très étalé, OH, échangeable avec $D_2O$; 11,8 ppm, 1 proton, dôme, $NH^+$, échangeable avec $D_2O$.

**Exemple 2**

Chlorhydrate de l'isopropyl-5 méthyl-2 phénylpipérazinyléthoxy-4 phénol ou COR 2837; chlorhydrate du produit de formule (I) avec
n = 0,
$R_2$ = phényle.

**Synthèse**

A une solution de 5,46 g de bromoéthoxy-4 isopropyl-5 méthyl-2 phénol dans 100 ml d'éthanol, on ajoute 2,5 équivalents de phénylpipérazine. Le mélange réactionnel est chauffé pendant 6 h au reflux. L'évolution de la réaction est suivie en chromatographie sur couches minces de silice avec comme solvant d'élution le mélange chloroforme 90/ méthanol 10 V/V. Après refroidissement, le bromhydrate de phénylpipérazine est filtré. Le filtrat est évaporé sous vide. Le résidu est repris par une solution diluée d'ammoniaque et extrait avec du chloroforme. La solution chloroformique est évaporée. Le résidu obtenu est repris par une solution diluée d'acide chlorhydrique. Après extraction au chloroforme, le précipité formé est filtré et lavé avec un peu d'éther éthylique puis est purifié par recristallisation. Rendement 56 %.

**Propriétes physicochimiques**

Point de fusion du chlorhydrate mesuré au banc Kofler : 190°C. Spectre de RMN du chlorhydrate dans le $DMSOD_6$ : 1,1 ppm, 6 protons, doublet, $C(CH_3)_2$; 2,1 ppm, 3 protons, singulet, $CH_3$ sur le cycle phényle; 3,0 - 3,9 ppm, 11 protons, massif complexe, 5 $CH_2$-N + -CH-; 4,4 ppm, 2 protons, triplet mal résolu, $OCH_2$; 6,6 - 7,5 ppm, 7 protons, massif complexe, protons aromatiques; 8,8 ppm, 1 proton, pic large, OH, échangeable avec $D_2O$; 11,7 ppm, 1 proton, dome, $NH^+$, echangeable avec $D_2O$.

**Exemple 3**

Chlorhydrate de l'isopropyl-5 méthyl-2 ((trifluorométhyl-3 phényl)pipérazinyléthoxy)-4 phénol ou COR 2839; chlorhydrate du produit de formule (I) dans laquelle
n = 0,
$R_2$ = trifluorométhyl-3 phényle.

**Synthèse**

A une solution de 5,46 g de bromoéthoxy-4 isopropyl-5 méthyl-2 phénol dans 100 ml d'éthanol, on ajoute 2,5 équivalents de trifluorométhyl-3 phénylpipérazine. Le mélange réactionnel est chauffé pendant 15 h au reflux. L'évolution de la réaction est suivie en chromatographie sur couches minces de silice avec comme solvant d'élution le mélange chloroforme 90/ méthanol 10 V/V. Après refroidissement, le bromhydrate de trifluorométhyl-3 phénylpipérazine est filtré. Le filtrat est évaporé sous vide. Le résidu est repris par une solution diluée d'ammoniaque et extrait avec du chloroforme. La solution chloroformique est évaporée. Le résidu obtenu est repris par une solution diluée d'acide chlorhydrique. Après extraction au chloroforme, le précipité formé est filtré et lavé avec un peu d'éther éthylique puis est purifié par recristallisation. Rendement 43 %.

**Propriétés physicochimiques**

Point de fusion du chlorhydrate mesuré au banc Kofler : 216°C. Spectre de RMN du chlorhydrate dans le $DMSOD_6$ : 1,2 ppm, 6 protons, doublet, $C(CH_3)_2$; 2,1 ppm, 3 protons, singulet, $CH_3$ sur le cycle phényle; 3,0 - 4,6 ppm, 13 protons, massif complexe, 6 $CH_2$ + -CH-; 6,6 - 7,7 ppm, 6 protons, massif complexe, protons aromatiques; 8,8 ppm, 1 proton, dôme, OH, échangeable avec $D_2O$; 11,8 ppm 1 proton, dôme $NH^+$, échangeable avec $D_2O$.

**Exemple 4**

Chlorhydrate de l'isopropyl-5 méthyl-2 (p-fluorophénylpipérazinyléthoxy)-4 phénol ou COR 2841; chlorhydrate du produit de formule (I) dans laquelle
n = 0,
$R_2$ = fluoro-4 phényle.

**Synthèse**

A une solution de 5,46 g de bromoéthoxy-4 isopropyl-5 méthyl-2 phénol dans 100 ml d'éthanol, on ajoute 2,5 équivalents de p-fluorophénylpipérazine. Le mélange réactionnel est chauffé pendant 15 h au reflux. L'évolution de la réaction est suivie en chromatographie sur couches minces de silice avec comme solvant d'élution le mélange chloroforme 90/ méthanol 10 V/V. Après refroidissement, le bromhydrate de p-fluorophénylpipérazine est filtré. Le filtrat est évaporé sous vide. Le résidu est repris par une solution diluée d'ammoniaque et extrait avec du chloroforme. La solution chloroformique est évaporée. Le résidu obtenu est repris par une solution diluée d'acide chlorhydrique. Après extraction au chloroforme, le précipité formé est filtré et lavé avec un peu d'éther éthylique puis est purifié par recristallisation. Rendement 60 %.

**Propriétés physicochimiques**

Point de fusion du chlorhydrate mesuré au banc Kofler : 227°C. Spectre de RMN du chlorhydrate dans le $DMSOD_6$ : 1,2 ppm, 6 protons, doublet, $C(CH_3)_2$; 2,1 ppm, 3 protons, singulet, $CH_3$ sur le cycle phényle; 2,9 - 4,7 ppm, 13 protons, massif complexe, 6 $CH_2$ + -CH-; 6,6 - 7,3 ppm, 6 protons, massif complexe, protons aromatiques; 8,8 ppm, 1 proton, pic étalé, OH, échangeable avec $D_2O$; 11,9 ppm, 1 proton, dôme, $NH^+$, échangeable avec $D_2O$.

**Exemple 5**

Chlorhydrate de l'isopropyl-5 méthyl-2 ((chloro-3 phényl)pipérazinyléthoxy)-4 phénol ou COR 2842; chlorhydrate du produit de formule (I) dans laquelle
n = 0,
$R_2$ = chloro-3 phényle.

**Synthèse**

A une solution de 5,46 g de bromoéthoxy-4 isopropyl-5 méthyl-2 phénol dans 100 ml d'éthanol, on ajoute 2,5 équivalents de m-chlorophénylpipérazine. Le mélange réactionnel est chauffé pendant 15 h au reflux. L'évolution de la réaction est suivie en chromatographie sur couches minces de silice avec comme solvant d'élution le mélange chloroforme 90/ méthanol 10 V/V. Après refroidissement, le bromhydrate de m-chlorophénylpipérazine est filtré. Le filtrat est évaporé sous vide. Le résidu est repris par une solution diluée d'ammoniaque et extrait avec du chloroforme. La solution chloroformique est évaporée. Le résidu obtenu est repris par une solution diluée d'acide chlorhydrique. Après extraction au chloroforme, le précipité formé est filtré et lavé avec un peu d'éther éthylique puis est purifié par recristallisation. Rendement 47 %.

**Propriétés physicochimiques**

Point de fusion du chlorhydrate mesuré au banc Kofler : 224°C. Spectre de RMN du chlorhydrate dans le $DMSOD_6$ : 1,2 ppm, 6 protons, doublet, $C(CH_3)_2$; 2,1 ppm, 3 protons, singulet, $CH_3$ sur le cycle phényle; 2,9 - 4,7 ppm, 13 protons, massif complexe, 6 $CH_2$ + -CH-; 6,6 - 7,4 ppm, 6 protons, massif complexe, protons aromatiques; 8,8 ppm, 1 proton, dôme, OH, échangeable avec $D_2O$; 12,0 ppm, 1 proton, dôme, NH, échangeable avec $D_2O$.

**Exemple 6**

Chlorhydrate de l'isopropyl-5 méthyl-2 (méthyl-2 phényl)pipérazinyléthoxy-4 phénol ou COR 2843; chlorhydrate du produit de formule (I) dans laquelle
n = 0,
$R_2$ = méthyl-2 phényle.

**Synthèse**

A une solution de 5,46 g de bromoéthoxy-4 isopropyl-5 méthyl-2 phénol dans 100 ml d'éthanol, on ajoute 2,5 équivalents de (méthyl-2 phényl)pipérazine. Le mélange réactionnel est chauffé pendant 15 h au reflux. L'évolution de la réaction est suivie en chromatographie sur couches minces de silice avec comme solvant d'élution le mélange chloroforme 90/ méthanol 10 V/V. Après refroidissement, le bromhydrate de (méthyl-2 phényl)pipérazine est filtré. Le filtrat est évaporé sous vide. Le résidu est repris par une solution diluée d'ammoniaque et extrait avec du chloroforme. La solution chloroformique est évaporée. Le résidu obtenu est repris par une solution diluée d'acide chlorhydrique. La solution obtenue est extraite au chloroforme; la phase chloroformique est lavée à l'eau, séchée, puis évaporée. Le résidu est lavé avec un peu d'éther éthylique puis est purifié par recristallisation. Rendement 74 %.

**Propriétés physicochimiques**

Point de fusion du chlorhydrate mesuré au banc Kofler : 209°C. Spectre de RMN du chlorhydrate dans le $DMSOD_6$ : 1,2 ppm, 6 protons, doublet, $C(CH_3)_2$; 2,1 ppm, 3 protons, singulet, $CH_3$ sur le cycle phénoxy; 2,3 ppm, 3 protons, singulet, $CH_3$ sur le cycle phénylpipérazine; 2,9 - 4,6 ppm, 13 protons, massif complexe, 6 $CH_2$ + -CH-; 6,6 - 7,4 ppm, 6 protons, massif complexe, protons aromatiques; 8,8 ppm, 1 proton, dôme, OH, échangeable avec $D_2O$; 11,9 ppm, 1 proton, dôme, $NH^+$, échangeable avec $D_2O$.

**Exemple 7**

Dichlorhydrate de l'isopropyl-5 méthyl-2 ((N-méthyl N-((méthoxy-2 phényl)pipérazinyléthyl)amino)-2 éthoxy)-4 phénol ou COR 2833; dichlorhydrate du produit de formule (I) avec
n = 1,
$R_1$ = $CH_3$,
$R_2$ = méthoxy-2 phényle.

**Synthèse**

On chauffe au reflux pendant 6 h une solution de 3,5 g de chloroéthyl-1 (méthoxy-2 phényl)-4 pipérazine et de 3 g d'isopropyl-5 méthyl-2 méthylaminoéthoxy-4 phénol dans 100 ml d'éthanol. La réaction est suivie en chromatographie sur couches minces (chloroforme/méthanol 10 %). Le solvant est évaporé. Le résidu est repris dans une solution aqueuse d'acide chlorhydrique. La solution obtenue est extraite au chloroforme. La phase aqueuse est alcalinisée avec de l'ammoniaque puis à nouveau extraite au chloroforme. Le chloroforme est séché et évaporé. Le produit brut est purifié par chromatographie sur colonne de silice, en éluant au chloroforme. La base est transformée en chlorhydrate par une solution d'éther éthylique saturée en acide chlorhydrique gazeux. Le précipité est filtré. Rendement 50 %.

**Propriétés physicochimiques**

Point de fusion du dichlorhydrate mesuré à l'appareil Mettler : 90° C. Spectre de RMN du dichlorhydrate dans le DMSOD$_6$ : 1,1 ppm, 6 protons, doublet, $C(CH_3)_2$; 2,1 ppm, 3 protons, singulet, $CH_3$ sur le cycle phényle; 2,8 - 4,1 ppm, 21 protons, massif complexe, 7 $CH_2$-N + $NCH_3$ à 3,0 ppm + -CH- + $OCH_3$ à 3,8 ppm; 4,3 ppm, 2 protons, triplet mal résolu, $OCH_2$ ; 6,6 - 7,2 ppm, 6 protons, massif complexe, protons aromatiques; 6 - 8,5 ppm, 2 protons, pic très étalé, protons labiles échangeables avec $D_2O$; 11,7 ppm, 1 proton, dôme, $NH^+$, échangeable avec $D_2O$.

**Exemple 8**

Dichlorhydrate de l'isopropyl-5 méthyl-2 ((N-méthyl N-(phénylpipérazinyléthyl)amino)-2 éthoxy)-4 phénol ou COR 2838; dichlorhydrate du produit de formule (I) avec
n = 1,
$R_1$ = $CH_3$,
$R_2$ = phényle.

**Synthèse**

On chauffe au reflux pendant 20 h une solution de 6 g d'isopropyl-5 méthyl-2 méthylaminoéthoxy-4 phénol et 6 g de chloroéthyl-1 phényl-4 pipérazine dans 10 ml de triéthylamine et 150 ml d'éthanol. L'éthanol et la triéthylamine en excès sont évaporés. Le résidu est repris par une solution aqueuse d'acide chlorhydrique. La solution obtenue est extraite au chloroforme. La phase aqueuse est alcalinisée avec de l'ammoniaque puis à nouveau extraite au chloroforme. Après avoir séché et évaporé le chloroforme on obtient 9,3 g de produit brut. Celui-ci est purifié par chromatographie sur colonne de silice, en éluant au chloroforme pur, puis avec un mélange chloroforme 98 % - méthanol 2 %. On obtient ainsi 4,6 g de base libre; rendement 42 %. Celle-ci est transformée en chlorhydrate par une solution d'éther éthylique saturée en acide chlorhydrique gazeux. On obtient ainsi un solide blanc qui est filtré et lavé avec un peu d'éther éthylique. Ce produit est hygroscopique et soluble dans l'eau.

**Propriétés physicochimiques**

Point de fusion du dichlorhydrate mesuré à l'appareil Mettler : 152° C. Spectre de RMN du dichlorhydrate dans le DMSOD$_6$ : 1,1 ppm, 6 protons, doublet, $C(CH_3)_2$; 2,1 ppm, 3 protons, singulet, $CH_3$ sur le cycle phényle; 2,8 - 4,6 ppm, 20 protons, massif complexe, 8 $CH_2$-N + $NCH_3$ à 3,0 ppm + -CH-; 6,6 - 7,6 ppm, 7 protons, massif complexe, protons aromatiques; 8,8 ppm, 1 proton, pic très étalé, OH, échangeable avec $D_2O$; 11,7 ppm, 2 protons, dôme, $NH^+$, échangeables avec $D_2O$.

**Exemple 9**

Dichlorhydrate de l'isopropyl-5 méthyl-2 ((méthoxy-4 phénylpipérazinyl)éthoxy)-4 phénol ou COR 2860; dichlorhydrate du produit de formule (I) dans laquelle
n = 0,
$R_2$ = méthoxy-4 phényle.

**Synthèse**

5,5 g de (bromo-2 éthoxy)-4 isopropyl-5 méthyl-2 phénol et 9,6 g de (méthoxy-4 phényl)pipérazine sont dissous dans 100 cm$^3$ d'éthanol. Cette solution est chauffée pendant 12 heures au reflux sous agitation. Le solvant est évaporé sous vide. Le résidu est alcalinisé avec une solution diluée d'ammoniaque, puis est extrait à l'aide de chloroforme. La solution chloroformique est séchée, le solvant est évaporé et le résidu est repris dans une solution diluée d'acide chlorhydrique puis extrait au chloroforme. La phase chloroformique est lavée en utilisant une solution diluée d'acide chlorhydrique, puis séchée et évaporée. Le produit brut ainsi obtenu est purifié par recristallisation dans un mélange de 40 ml de méthanol et 120 ml d'éther éthylique. Rendement 42 %.

**Propriétés physicochimiques**

Point de fusion du dichlorhydrate mesuré à l'appareil Mettler : 181,9 - 182,8°C. Spectre de RMN du dichlorhydrate dans le $DMSOD_6$ : 1,1 ppm, 6 protons, doublet, $C(CH_3)_2$; 2,1 ppm, 3 protons, singulet, $CH_3$ sur le cycle phénolique; 2,9 - 4,1 ppm, 14 protons, massif complexe, 5 $CH_2$-N + -CH- + $OCH_3$ à 3,7 ppm; 4,4 ppm, 2 protons, triplet mal résolu, $OCH_2$; 6,7 et 6,8 ppm, 2 protons, singulets, protons aromatiques du cycle phénol; 7,1 ppm, 4 protons, septème AA''BB', protons aromatiques du cycle phénylpipérazine; 9,5 - 12,7 ppm 3 protons, pics très étalés, protons labiles; échangeables avec $D_2O$.

**Exemple 10**

Chlorhydrate de l'isopropyl-5 méthyl-2 ((diméthyl-2,3 phénylpipérazinyl)éthoxy)-4 phénol ou COR 2861; chlorhydrate du produit de formule (I) avec
n = 0,
$R_2$ = diméthyl-2,3 phényle.

**Synthèse**

Le mélange constitué par 8,19 g de bromoéthoxy-4 isopropyl-5 méthyl-2 phénol, 3,8 g de (diméthyl-2,3 phényl)pipérazine, 100 ml d'éthanol et 40 ml de triéthylamine est chauffé pendant 12 h au reflux. Le mélange réactionnel est ensuite évaporé sous vide et le résidu est repris par une solution diluée d'ammoniaque puis extrait avec du chloroforme. La solution chloroformique est séchée et évaporée. Le résidu obtenu est repris par une solution diluée d'acide chlorhydrique puis extrait au chloroforme; la phase chloroformique est lavée avec une solution diluée d'acide chlorhydrique et séchée; le chloroforme est évaporé. Le résidu liquide obtenu est trituré dans de l'éther éthylique. Le solide brut obtenu est purifié par recristallisation dans 35 ml de méthanol. Rendement 49 %.

**Propriétés physicochimiques**

Point de fusion du chlorhydrate mesuré à l'appareil Mettler : 234°C. Spectre de RMN du chlorhydrate dans le $DMSOD_6$ : 1,1 ppm, 6 protons, doublet, $C(CH_3)_2$; 2,1 - 2,2 - 2,23 ppm, 9 protons, singulets, 3 $CH_3$ sur les cycles phényle; 2,9 - 4,0 ppm, 11 protons, massif complexe, 5 $CH_2$-N + -CH-; 4,4 ppm, 2 protons, triplet mal résolu, $OCH_2$; 6,7 et 6,8 ppm, 2 protons, singulets, protons aromatiques du cycle phénolique; 6,8 - 7,2 ppm, 3 protons, massif complexe, protons aromatiques du cycle phénylpipérazine; 8,9 ppm, 1 proton, dôme, OH, échangeable avec $D_2O$; 11,9 ppm, 1 proton, dôme, $NH^+$, échangeable avec $D_2O$.

**Exemple 11**

Chlorhydrate de l'isopropyl-5 méthyl-2 ((chloro-2 phényl) pipérazinyléthoxy)-4 phénol ou COR 2864; chlorhydrate du produit de formule (I) dans laquelle
n = 0,
$R_2$ = chloro-2 phényle.

**Synthèse**

Le mélange constitué par 8,19 g de bromoéthoxy-4 isopropyl-5 méthyl-2 phénol, 3,93 g de (chloro-2 phényl) pipérazine, 100 ml d'éthanol et 40 ml de triéthylamine est chauffé pendant 12 h au reflux. Le mélange réactionnel est ensuite évaporé sous vide et le résidu est repris par une solution diluée d'ammoniaque puis extrait au chloroforme. La solution chloroformique est séchée et évaporée. Le résidu obtenu est repris par une solution diluée d'acide chlorhydrique puis extrait au chloroforme; la phase chloroformique est lavée à l'eau et séchée sur sulfate de sodium anhydre; le chloroforme est évaporé. Le solide obtenu est trituré dans de l'éther éthylique pour éliminer l'excès de dérivé bromé, puis purifié par recristallisation dans un mélange méthanol - éther éthylique. Rendement 26 %.

**Propriétés physicochimiques**

Point de fusion du chlorhydrate mesuré à l'appareil Mettler : 205,2° C . Spectre de RMN du chlorhydrate dans le $DMSOD_6$ : 1,1 ppm, 6 protons, doublet, $C(CH_3)_2$; 2,1 ppm, 3 protons, singulet, $CH_3$ sur le cycle phénolique; 2,9 - 4,0 ppm, 11 protons, massif complexe, 5 $CH_2$ N + -CH-; 4,4 ppm, 2 protons, triplet mal résolu, $OCH_2$; 6,7 et 6,8 ppm, 2 protons, singulets, protons aromatiques du cycle phénolique; 6,9 - 7,7 ppm, 4 protons, massif complexe, protons aromatiques phénylpipéraziniques; 8,8 ppm, 1 proton, dôme, OH, échangeable avec $D_2O$; 11,5 ppm, 1 proton, dôme, $NH^+$, échangeable avec $D_2O$.

**Exemple 12**

Chlorhydrate de l'isopropyl-5 méthyl-2 ((éthoxy-2 phényl)pipérazinyléthoxy)-4 phénol ou COR 2865; chlorhydrate du produit de formule (I) dans laquelle
n = 0,
$R_2$ = éthoxy-2 phényle.

**Synthèse**

On chauffe au reflux de l'éthanol pendant 6 heures, sous agitation, le mélange constitué par 8 g de (bromo-2 éthoxy)-4 isopropyl-5 méthyl-2 phénol, 6 g d'(éthoxy-2 phényl)-4 pipérazine, 100 ml d'éthanol et 5 ml de triéthylamine. La réaction est suivie en chromatographie sur couches minces en éluant par le mélange méthanol/chloroforme 10/90 V/V. Les solvants sont évaporés. On ajoute au résidu d'évaporation 150 ml d'acide chlorhydrique 1N; le mélange est soumis à une forte agitation. La phase aqueuse contenant l'(éthoxy-2 phényl)-4 pipérazine est éliminée. Le produit pâteux est dissous dans du méthanol; quelques gouttes d'acide chlorhydrique concentré sont ajoutées à la solution méthanolique qui est concentrée au Rotavapor. De l'éther éthylique est ajouté afin de faire précipiter le produit qui est filtré et redissous dans du méthanol. On neutralise en utilisant de l'ammoniaque et le solvant est éliminé par évaporation. Le résidu d'évaporation est dissous dans du chloroforme; la solution est lavée deux fois à l'eau, séchée sur sulfate de sodium. Le chloroforme est éliminé par évaporation. Le produit obtenu est purifié sur colonne de silice en éluant d'abord au chloroforme puis au mélange méthanol/chloroforme à 3 puis 5 %. Le produit obtenu est dissous dans de l'éther éthylique. Il est transformé en chlorhydrate par addition d'éther éthylique saturé en acide chlorhydrique gazeux. Le précipité est filtré puis séché. Rendement 52 %.

**Propriétés physicochimiques**

Point de fusion mesuré à l'appareil Mettler : 233,1 - 233,6°C. Spectre de RMN du chlorhydrate dans le $DMSOD_6$ : 1,1 ppm, 6 protons, doublet, $C(CH_3)_2$; 1,4 ppm, 3 protons, triplet, $CH_3$ du groupement éthoxy; 2,1 ppm, 3 protons, singulet, $CH_3$ sur le cycle phénolique; 2,9 - 4,7 ppm, 15 protons, massif complexe, 7 $CH_2$ + -CH- ; 6,6 - 7,1 ppm, 6 protons, massif complexe, protons aromatiques; 8,8 ppm, 1 proton, dôme, OH, échangeable avec $D_2O$; 11,7 ppm, 1 proton, dôme, $NH^+$, échangeable avec $D_2O$.

**Exemple 13**

Dichlorhydrate de l'isopropyl-5 méthyl-2 ((pyridyl-2)pipérazinyléthoxy)-4 phénol ou COR 2873; dichlorhydrate du produit de formule (I) dans laquelle
n = 0,
$R_2$ = pyridyl-2.

**Synthèse**

Le mélange constitué par 8,1 g de bromoéthoxy-4 isopropyl-5 méthyl-2 phénol, 15 g de pyridyl-2 pipérazine et 100 ml d'éthanol est chauffé sous agitation pendant 8 h au reflux. Le précipité est éliminé par filtration. Le filtrat est évaporé sous vide. Le résidu d'évaporation est repris par de l'éther éthylique et extrait par une solution chlorhydrique. La phase aqueuse acide est alcalinisée, puis extraite au chloroforme. La phase chloroformique est lavée à l'eau, séchée sur sulfate de sodium et évaporée. Le résidu d'évaporation est

dissous dans le minimum de méthanol. De l'éther éthylique est rajouté et la solution est saturée en acide chlorhydrique gazeux. Le précipité formé est filtré et lavé avec de l'éther éthylique. Rendement 38 %.

**Propriétés physicochimiques**

Point de fusion du dichlorhydrate mesuré à l'appareil Mettler : 213,7 - 215,2°C. Spectre de RMN du dichlorhydrate dans le $DMSOD_6$ : 1,1 ppm, 6 protons, doublet, $C(CH_3)_2$; 2,1 ppm, 3 protons, singulet, $CH_3$ sur le cycle phényle; 2,9 - 5,2 ppm, 13 protons, massif complexe, 6 $CH_2$ + -CH-; 6,6 - 8,3 ppm, 6 protons, massif complexe, protons aromatiques; 10,1 - 13,2 ppm, 3 protons, pics étalés, OH + 2 $NH^+$, échangeables avec $D_2O$.

**Exemple 14**

Chlorhydrate de l'isopropyl-5 méthyl-2 (propoxy-2 phényl)pipérazinyléthoxy-4 phénol ou COR 2876; chlorhydrate du produit de formule (I) dans laquelle
n = 0,
$R_2$ = propoxy-2 phényle.

**Synthèse**

Le mélange constitué par 15 g de bromoéthoxy-4 isopropyl-5 méthyl-2 phénol, 200 ml d'éthanol, 8 g de (propoxy-2 phényl)pipérazine et 20 ml de triéthylamine est chauffé pendant une nuit au reflux. Le solvant est éliminé par évaporation. Le résidu est dissous dans du chloroforme. La solution est extraite deux fois à l'acide chlorhydrique 1 N pour éliminer la (propoxy-2 phényl)pipérazine qui n'a pas réagi. La solution chloroformique est séchée, puis évaporée. Le résidu obtenu est repris par de l'éther, puis filtré. La phase solide est solubilisée dans du méthanol. La solution est neutralisée par de l'ammoniaque jusqu'à pH basique puis acidifiée par de l'acide chlorhydrique concentré. Le solvant est éliminé par évaporation. Le résidu est dissous dans du chloroforme; la phase chloroformique est lavée à l'eau, séchée, puis évaporée. Le résidu est lavé avec un peu d'éther éthylique puis est filtré. Rendement 73 %.

**Propriétés physicochimiques**

Point de fusion du chlorhydrate mesuré à l'appareil Mettler : 223,2 - 223,6°C. Spectre de RMN du chlorhydrate dans le $DMSOD_6$ : 0,8 - 1,4 ppm, 9 protons, massif complexe, $C(CH_3)_2$ + $CH_3$ du groupement propoxy; 1,8 ppm, 2 protons, multiplet, $CCH_2C$ du groupement propoxy; 2,1 ppm, 3 protons, singulet, $CH_3$ sur le cycle phénolique; 2,9 - 4,7 ppm, 15 protons, massif complexe, 7 $CH_2$ (pipérazine + 2 $OCH_2C$ + $NCH_2C$ ) + -CH- ; 6,6 - 7,2 ppm, 6 protons, massif complexe, protons aromatiques; 6 - 9 ppm, 1 proton, pic très étalé, OH, échangeable avec $D_2O$; 12 ppm, 1 proton, dôme, $NH^+$, échangeable avec $D_2O$.

**Exemple 15**

Chlorhydrate de l'isopropyl-5 méthyl-2 (hydroxy-2 phényl)pipérazinyléthoxy-4 phénol ou COR 2877; chlorhydrate du produit de formule (I) dans laquelle
n = 0,
$R_2$ = hydroxy-2 phényle.

**Synthèse**

Le mélange constitué par 20 g de bromoéthoxy-4 isopropyl-5 méthyl-2 phénol, 150 ml d'éthanol, 10 g d'(hydroxy-2 phényl)pipérazine et 20 ml de triéthylamine est chauffé pendant 12 h au reflux. Le solvant est éliminé par évaporation. De l'eau puis de l'ammoniaque concentré sont ajoutés au résidu; on procède à deux extractions au chloroforme. La solution chloroformique est séchée, puis évaporée. Le résidu obtenu est repris par 50 ml de méthanol. On fait passer un courant d'acide chlorhydrique gazeux dans la solution de manière à synthétiser le chlorhydrate. Après refroidissement, le précipité est filtré, lavé au méthanol puis à l'éther

éthylique. Rendement 68 %.

### Propriétés physicochimiques

Point de fusion du chlorhydrate mesuré à l'appareil Mettler : 279,1 - 279,3°C . Spectre de RMN du chlorhydrate dans le $DMSOD_6$ : 1,1 ppm, 6 protons, doublet, $C(CH_3)_2$; 2,1 ppm, 3 protons, singulet, $CH_3$ sur le cycle phénoxy; 2,8 - 4,0 ppm, 11 protons, massif complexe, 5 $CH_2$ + -CH-; 4,4 ppm, 2 protons, triplet mal résolu, $OCH_2$; 6,6 - 7,1 ppm, 6 protons, massif complexe, protons aromatiques; 8,9 et 9,4 ppm, 2 protons, pics larges, 2 OH, échangeables avec $D_2O$; 11,5 ppm, 1 proton, pic très étalé, $NH^+$, échangeable avec $D_2O$.

Les propriétés toxicopharmacologiques des produits faisant l'objet de la présente invention vont être exposées ci-après.

### Pharmacotoxicologie

Toxicité : Administrés à la souris par voie orale à la dose de 300 mg/kg, les COR 2831, COR 2837, COR 2839, COR 2841, COR 2842, COR 2843, COR 2860, COR 2861, COR 2864 n'entraînent aucune mortalité; chez cet animal et par cette voie d'administration, la DL50 du COR 2831 est de 1130 (1026 - 1246) mg/kg et celle du COR 2865 est de 1444 (1212 - 1719) mg/kg.

Administrés à la souris par voie intrapéritonéale, les COR 2831, COR 2837, COR 2839, COR 2841, COR 2842, COR 2843, COR 2860, COR 2861, COR 2864, COR 2865 utilisés à la dose de 200 mg/kg et le COR 2838 à la dose de 50 mg/kg n'entraînent aucune mortalité.

Administrés à la souris par voie intraveineuse, le COR 2831 présente une DL50 de 86 (76,6 - 98) mg/kg et le COR 2865 entraîne 0 % de mortalité à 6,2 mg/kg, 10 % à 12,5 mg/kg, 20 % à 25 mg/kg, 5 % à 50 mg/kg et 90 % à 100 mg/kg.

Activité alpha-1 bloquante : In vitro l'activité alpha-1 bloquante est appréciée par la détermination de l'antagonisme des contractions de strips aortiques isolés de lapin induites par la noradrénaline employée à la concentration de $2{,}10^{-6}$ mole/l selon une technique dérivée de celle de Furchgott et Bhadrakom (J. Pharmacol., 1953, 108, 129 - 43). On donne ci-après pour les dérivés faisant l'objet de la présente invention, les CI50 (concentrations inhibant 50 % des contractions) ainsi que le délai entre l'introduction du produit à tester et l'introduction de l'agoniste dans le bain, ce délai correspondant à l'activité maximale : COR 2831, 90 mn, $1{,}10.10^{-7}$ ($9{,}26.10^{-8}$ - $1{,}31.10^{-7}$) mole/l; COR 2837, 30 mn, $1{,}64.10^{-6}$ ($1{,}44.10^{-6}$ - $1{,}85.10^{-6}$) mole/l; COR 2841, 60 mn, $1{,}16\text{-}10^{-6}$ ($9{,}24.10^{-7}$ - $1{,}46.10^{-6}$) mole/l; COR 2843, 60 mn, $9{,}82.10^{-7}$ ($7{,}67.10^{-7}$ - $1{,}26.10^{-6}$) mole/l; COR 2838, 60 mn, $2{,}53\ 10^{-6}$ ($2{,}15\ 10^{-6}$ - $2{,}98\ 10^{-6}$) mole/l. Le type d'antagonisme du COR 2831 vis à vis de la noradrénaline a été déterminé sur le même type de préparation, pour un délai de 30 mn en utilisant la méthode des courbes cumulatives de Van Rossum en présence de cocaïne; il est de nature compétitive, le $pA_2$ calculé selon la méthode d'Arunlakshama et Schild étant de 8,59 ± 0,08. In vivo, l'activité alphabloquante est déterminée chez la souris dans le test de l'inhibition de la mydriase induite par la norépinéphrine. Les animaux reçoivent le produit à tester par voie intrapéritonéale; 30 mn plus tard, ils reçoivent 0,75 mg/kg de norépinéphrine par voie intraveineuse. Dans ces conditions, le COR 2865, produit non cholinergique, administré aux doses de 2,5 et 1 mg/kg, entraîne respectivement 80 et 54 % d'inhibition de la mydriase, alors que le labétalol entraîne 58 % d'inhibition à 25 mg/kg.

In vivo l'activité alpha bloquante est également appréciée par la détermination de l'antagonisme de l'hypertension induite par la phényléphrine. Les produits à tester sont administrés par voie intraveineuse à des rats amyélés et bivagotomisés selon la technique de J.S. Gillespie et T.S. Muir (Br. J. Pharmac. Chemother., 1967, 30, 78 - 87). On indique ci-après la dose administrée, l'activité maximale enregistrée et le temps au bout duquel l'activité résiduelle est égale à 50 % de l'activité maximale : COR 2831 ($3.10^{-6}$ M/kg - 129,2 % - > 2h); COR 2837 ($3.10^{-6}$ M/kg - 91,1 % - 15 mn); COR 2841 ($3.10^{-6}$ M/kg - 39,8 % - 15 mn); COR 2842 ($3.10^{-6}$ M/kg - 35,1 % - 15 mn), COR 2843 ($3.10^{-6}$ M/kg - 112,3 % - 25 mn); COR 2838 ($3.10^{-6}$ M/kg - 60 % - 5 mn), COR 2860 ($3.10^{-6}$ M/kg - 39,7 % - 4 mn); COR 2861 ($3.10^{-6}$ M/kg - 66,4 % - 9 mn); COR 2864 ($3.10^{-6}$ M/kg - 98,6 % - 37 mn; COR 2865 ($10^{-6}$ M/kg - 121,8 % - 109 mn; $3.10^{-7}$ M/kg - 116,1 % - 32 mn). La DE50 déterminé dans ces conditions pour le COR 2831 est de $1\ 95.10^{-8}$ mole/kg au bout de 2 mn, $3{,}04.10^{-7}$ mole/kg au bout de 30 mn et $8{,}4.10^{-7}$ mole/kg au bout de 60 mn; pour le COR 2865 elle est de $1{,}17.10^{-8}$ mole/kg au bout de 2 mn, $3{,}18.10^{-7}$ mole/kg au bout de 30 mn et $5{,}0.10^{-7}$ mole/kg au bout de 60 mn.

Activité antihypertensive : les produits à tester sont administrés par voie orale à des rats spontanément hypertendus. La pression artérielle est mesurée de façon indirecte en utilisant un sphygmomanomètre. Pour le COR 2831 administré à la dose de 100 mg/kg/j pendant 2 jours, la chute de pression maximale observée est de respectivement 26 et 21 % le premier et le deuxième jour de traitement, une heure après le gavage.

Dans une autre expérience, réalisée également par voie orale et chez le rat spontanément hypertendu, la pression artérielle est mesurée de façon indirecte sur la queue 2, 4, 6 heures après l'administration du produit. Pour le COR 2865 administré à la dose de 100 mg/kg la réduction de pression est respectivement de 19, 21, 23

% à ces échéances.

Activité hypoglycémiante : le COR 2839 administré à la dose de 100 mg/kg par voie orale à la souris entraîne au bout d'une heure 21 % de réduction de la courbe de tolérance du glucose après surcharge par l'amidon (2,5 g/kg). 1 heure après administration orale à la dose de 100 mg/kg, le COR 2839 entraîne 24 % de diminution de la glycémie chez des souris à jeun soumises à une surcharge en glucose (1 g/kg) au moment de l'administration du produit à tester.

Activité anticalcique : sur oreillette gauche de cobaye stimulée électriquement à 150 battements/mn dans une solution de Tyrode pauvre en calcium (0,6 mM) le COR 2835 employé à $25.10^{-6}$ g/ml inhibe de 50 % l'augmentation de la force de contraction induite par l'addition de 0,6 mM de calcium.
Le COR 2838 employé à $5.10^{-6}$ g/ml inhibe l'augmentation, induite par le calcium, des contractions spontanées de strips de veines mésentériques porte de rats normotendus.
Le COR 2838 inhibe à 50 % les contractions d'aorte isolée de lapin induites par $60.10^{-6}$ M de KCl à la concentration de $9{,}44.10^{-7}$ M/l selon la technique de Karaki, Br. J. Pharmac. 1982, 77, 661 - 666).

Activité antiallergique - antihistaminique : dans le test de l'anaphylaxie passive cutanée, les COR 2833 et COR 2838 administrés à 10 mg/kg par voie IV chez le rat entraînent respectivement 53 et 60 % d'activité. Le COR 2831 entraîne dans les mêmes conditions 67 % d'activité à la dose de 5 mg/kg administrée par voie orale.
Les COR 2831 et COR 2838 utilisés in vitro à la concentration de $10^{-5}$ g/ml et le COR 2833 à la concentration de $2.10^{-6}$ g/ml inhibent à 80 % la contraction induite par $5.10^{-7}$ g/ml d'histamine sur iléon isolé de cobaye; quand les contractions de l'iléon sont induites par $10^{-8}$ g/ml d'histamine et que le produit à tester est introduit dans le bain 10 minutes avant l'histamine, le COR 2831 présente une CI50 de $3{,}26.10^{-6}$ ($2{,}68.10^{-6}$ - $4{,}03.10^{-6}$) M/1.
L'activité antihistaminique-antiallergique a été également déterminée en couplant sur le même animal (rat Sprague-Dawley) une réaction anaphylactique (IgE) et une réaction cutanée à l'histamine. L'activité est évaluée par la quantité de bleu d'Evans libérée au niveau de chaque site réactionnel comparativement à des animaux témoins recevant le véhicule d'administration. Le COR 2831 entraîne dans ce test les DE50 suivantes:
voie orale IgE 14,4 (7,7 - 27) mg/kg
histamine 8,0 (4,3 - 15) mg/kg
voie intraveineuse IgE 0,27 (0,15 - 0,40) mg/kg
histamine 0,37 (0,21 - 0,62) mg/kg

Activité antisérotonine : le COR 2838 utilisé in vitro à la concentration de $4.10^{-7}$ g/ml et les COR 2831 et COR 2833 à la concentration de $2.10^{-6}$ g/ml inhibent les contractions induites par 10 g/ml de sérotonine sur iléon isolé de cobaye; quand les contractions de l'iléon sont induites par $5.10^{-7}$ g/ml de sérotonine et que le produit à tester est introduit dans le bain 15 minutes avant l'agoniste, le COR 2831 présente une CI50 de 2,74 (1,59-3,99).$10^{-6}$ M/1.

L'activité neuroleptique du COR 2865 a été déterminée dans le test du "climbing" des souris présélectionnées placées dans des cages aménagées spécialement pour le test reçoivent le produit à tester par voie intrapéritonéale et leur comportement de climbing est coté de 0 à 2 dans les 30 mn qui suivent l'injection. Les animaux ne présentant pas d'effet agoniste significatif, on leur administre 1,5 mg/kg d'apomorphine de façon à induire un climbing de côte maximale. Dans ces conditions, le COR 2865 entraîne, aux doses de 25 et 10 mg/kg respectivement 100 et 83 % d'inhibition du climbing induit par l'apomorphine.
L'activité neuroleptique a également été mise en évidence dans le test de l'inhibition des stéréotypies induites par l'apomorphine chez le rat; les animaux reçoivent le produit à tester par voie intrapéritonéale puis, 30 mn plus tard, 2,5 mg/kg d'apomorphine par voie sous-cutanée; leur comportement est noté pendant les 30 mn qui suivent. Dans ces conditions, le COR 2865, administré à la dose de 100 mg/kg, inhibe de 67 % les stéréotypies induites par l'apomorphine.

Activité hypolipidémiante : chez la souris rendue hypercholestérolémique par un régime riche en cholestérol et acide cholique pendant 7 jours, l'administration de 100 mg/kg de COR 2865 par voie orale, le 6° et le 7° jour, entraîne une diminution du cholestérol sérique de 31 % et une diminution des HP-bétalipoprotéines de 38 % accompagnées d'une diminution du rapport VLDL + LDL / cholestérol.

Activité antiagrérante plaquettaire : l'agrégation plaquettaire induite par 50 microg/ml d'arachidonate de sodium en présence ou non de 1 microg/ml d'aspirine, dans un plasma de lapin riche en plaquettes est inhibée par les produits de la présente invention; ainsi en présence d'aspirine, cette inhibition est de 100 % aux concentrations de 2,5 microg/ml pour le COR 2860 et 5 microg/ml pour le COR 2864. En l'absence d'aspirine, cette inhibition est de 100 % aux concentrations de 5 microg/ml pour le COR 2860 et de 10 microg/ml pour le COR 2864.
Compte tenu de leur faible toxicité et de leurs propriétés pharmacologiques et en particulier de leurs propriétés alpha-1 bloquantes et antihypertensives, les produits faisant l'objet de la présente invention peuvent être employés par exemple dans le traitement de l'hypertension artérielle (seuls ou en association avec un diurétique ou d'autres médicaments antihypertenseurs), dans le traitement des troubles vasculaires

périphériques tels que l'acrocyanose et le syndrôme de Raynaud, dans le traitement du glaucome.

Compte tenu de leur activité antiagrégante plaquettaire, les produits faisant l'objet de la présente invention sont utiles dans le traitement au long cours après infarctus du myocarde.

Compte tenu de leur activité hypolipidémiante, les produits faisant l'objet de la présente invention sont utiles dans le traitement des hyperlipémies.

Compte tenu de leur activité neuroleptique, les produits faisant l'objet de la présente invention sont utiles dans le traitement des psychoses, des états anxieux et des états d'agressivité.

Compte-tenu de leurs propriétés anticalciques, les produits de la présente invention peuvent être employés par exemple dans le traitement de l'angor et des troubles du rythme cardiaque.

Compte tenu de leurs propriétés antiallergiques et antihistaminiques, les produits de la présente invention peuvent être utilisés dans le traitement des états allergiques.

Les doses et schémas thérapeutiques seront fonction du sujet et de l'affection à traiter. Les produits pourront être administrés par voie orale (par exemple sous forme de gélules, comprimés, gouttes buvables), par voie injectable (soluté injectable pour la voie intramusculaire ou la voie intraveineuse; soluté pour perfusion intraveineuse), par voie rectale (suppositoires), par voie locale (collyres pour le traitment du glaucome, crèmes, pommades, gels). Suivant les indications, la dose quotidienne variera de 1 à 100 mg en 1 à 3 prises pour la voie orale, de 1 à 100 mg en 1 ou 2 prises pour la voie rectale; la dose administrée par voie intraveineuse pourra varier entre 0,1 et 10 mg. Les collyres contiendront 0,05 à 0,5 % de principe actif et les pommades, crèmes ou gels contiendront 0,5 à 5 % de principe actif.

## Revendications

1. Produits de formule générale (I)

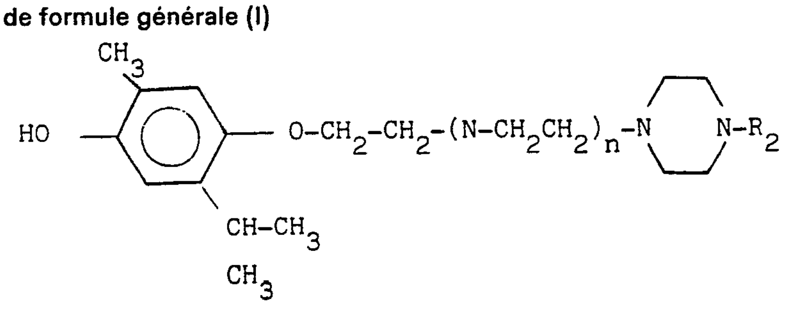

(I)

dans laquelle

n est égal à 0 ou 1,

$R_1$ représente un atome d'hydrogène ou un groupement méthyle, et

$R_2$ represente soit un cycle phényle substitue ou non par un ou plusieurs substituants semblables ou différents choisis dans le groupe hydroxy alcoxy de $C_1$ à $C_3$ (par exemple méthoxy, éthoxy, propoxy), trifluorométhyle, chloro, fluoro, ou alkyle $C_1$ et $C_2$ (par exemple méthyle ou éthyle), soit un cycle pyridyl-2, -3 ou -4,

sous forme de base libre ou des sels d'addition pharmaceutiquement compatibles.

2. Produits selon la revendication 1 caractérisés en ce que n = 0.

3. Produits selon la revendication 1 caractérisés en ce que n = 1.

4. Méthode de préparation des produits selon la revendication 1 caractérisée en ce que l'on fait réagir un (halogéno-2 éthoxy)-4 isopropyl-5 méthyl-2 phénol et une arylpipérazine de formule générale (IIa) dans laquelle

n est égal à 0 ou 1,

$R^1$ représente un atome d'hydrogène ou un groupement méthyle et

$R_2$ représente soit un cycle phényle substitué ou non par un ou plusieurs substituants semblables ou différents choisis dans le groupe hydroxy, alcoxy de $C_1$ à $C_3$ (par exemple méthoxy, éthoxy, propoxy), trifluorométhyle, chloro, fluoro, ou alkyle de $C_1$ et $C_2$ (par exemple méthyle ou éthyle), soit un cycle pyridyl-2, -3 ou -4, le terme halogéno représentant un atome de chlore ou de brome.

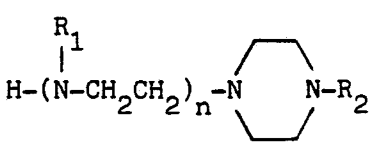

(II)

5. Méthode de préparation des produits selon la revendication 3 caractérisée en ce que l'on fait réagir une halogénoéthyl-1 aryl-4 pipérazine de formule

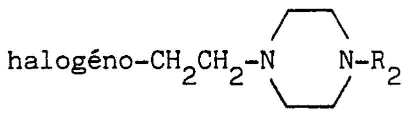

(III)

générale (III) dans laquelle

le terme halogéno représente un atome de brome ou de chlore et

$R_2$ représente soit un cycle phényle substitué ou non par un ou plusieurs substituants semblables ou différents choisis dans le groupe hydroxy, alcoxy de $C_1$ à $C_3$ (par exemple méthoxy, éthoxy, propoxy), trifluorométhyle, chloro, fluoro, ou alkyle de $C_1$ et $C_2$ (par exemple méthyle ou éthyle), soit un cycle pyridyl-2, -3 ou -4 et un isopropyl-5 méthyl-2 aminoéthoxy-4 phénol

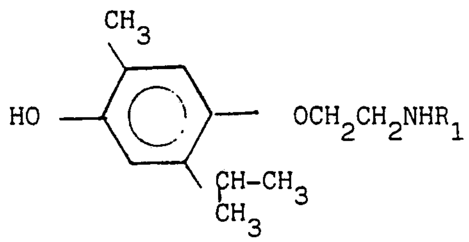

(IV)

de formule générale (IV) avec $R_1$ = H ou méthyle.

6. Médicaments utiles en thérapeutique humaine ou vétérinaire contenant une quantité thérapeutiquement efficace d'au moins un composé selon les revendications 1 à 3.

7. Médicaments utiles en thérapeutique cardiovasculaire contenant une quantité thérapeutiquement efficace d'au moins un composé selon les revendications 1 à 3.

8. Médicaments utiles en thérapeutique antiallergique contenant une quantité thérapeutiquement efficace d'au moins un composé selon les revendications 1 à 3.

9. Médicaments utiles en thérapeutique psychotrope contenant une quantité thérapeutiquement efficace d'au moins un composé selon les revendications 1 à 3.

10. Médicaments utiles en thérapeutique hypolipidémiante contenant une quantité thérapeutiquement efficace d'au moins un composé selon les revendications 1 à 3.

11. Composition pharmaceutique ou vétérinaire caractérisée en ce qu'elle contient à titre de principe actif au moins un produit selon les revendications 1 à 3 en association avec un véhicule pharmaceutique ou un excipient approprié.

## Patentansprüche

1. Verbindungen der allgemeinen Formel (I)

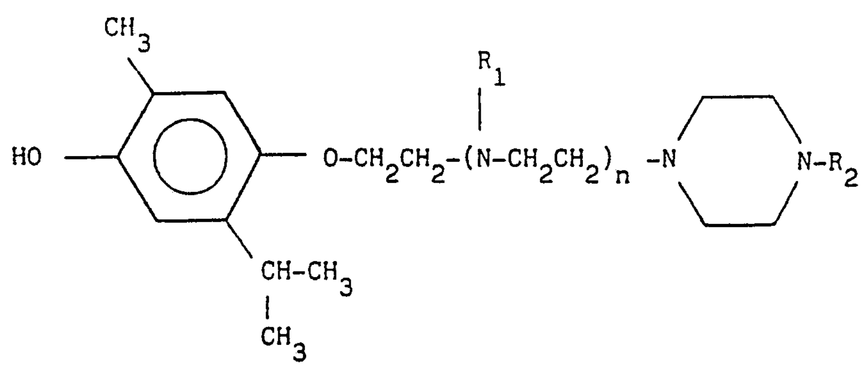

(I)

in der

n die Zahl 0 oder 1,

$R_1$ ein Wasserstoffatom oder eine Methylgruppe und

$R_2$ entweder einen Phenylring, der gegebenenfalls mit einem oder mehreren gleichen oder verschiedenen Substituenten aus der von Hydroxy, $C_1$-$C_3$-Alkoxy (z. B. Methoxy, Ethoxy, Propoxy), Trifluormethyl, Chlor, Fluor und $C_1$-$C_2$-Alkyl (z. B. Methyl oder Ethyl) gebildeten Gruppe substituiert ist, oder einen Pyridyl-2-, -3- oder -4-Ring bedeuten,

in Form der freien Basen oder der pharmazeutisch verträglichen Additionssalze.

2. Verbindungen nach Anspruch 1, dadurch gekennzeichnet, daß n = 0 ist.

3. Verbindungen nach Anspruch 1, dadurch gekennzeichnet, daß n = 1 ist.

4. Verfahren zur Herstellung der Verbindungen nach Anspruch 1, dadurch gekennzeichnet, daß man ein (Halogen-2-ethoxy)-4-isopropyl-5-methyl-2-phenol und ein Arylpiperazin der allgemeinen Formel (IIa), in der

n die Zahl 0 oder 1,

$R_1$ ein Wasserstoffatom oder eine Methylgruppe und

$R_2$ entweder einen Phenylring, der gegebenenfalls mit einem oder mehreren gleichen oder verschiedenen Substituenten aus der von Hydroxy, $C_1$-$C_3$-Alkoxy (z. B. Methoxy, Ethoxy, Propoxy), Trifluormethyl, Chlor, Fluor und $C_1$-$C_2$-Alkyl (z. B. Methyl oder Ethyl) gebildeten Gruppe substituiert ist, oder einen Pyridyl-2-, -3- oder -4-Ring bedeuten und der Begriff Halogen ein Chlor- oder Bromatom bedeuten.

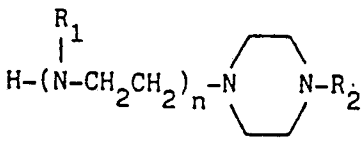

(IIa)

5. Verfahren zur Herstellung der Verbindungen nach Anspruch 3, dadurch gekennzeichnet, daß man ein Halogenethyl-1-aryl-4-piperazin der Formel

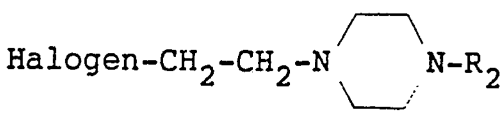

(III)

in der $R_2$ entweder einen Phenylring, der gegebenenfalls mit einem oder mehreren gleichen oder verschiedenen Substituenten aus der von Hydroxy, $C_1$-$C_3$-Alkoxy (z. B. Methoxy, Ethoxy, Propoxy), Trifluormethyl, Chlor, Fluor und $C_1$-$C_2$-Alkyl (z. B. Methyl oder Ethyl) gebildeten Gruppe substituiert ist, oder einen Pyridyl-2-, -3- oder -4-Ring bedeuten, mit einem Isopropyl-5-methyl-2-aminoethoxy-4-phenol der allgemeinen Formel

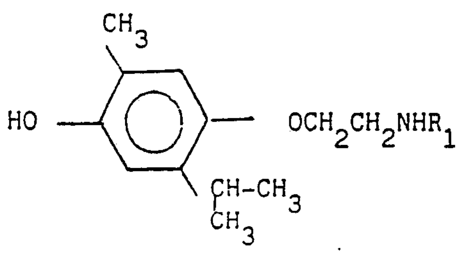

(IV)

in der $R_1$ Wasserstoff oder Methyl bedeutet, umsetzt.

6. Medikamente zur Verwendung in der menschlichen oder veterinärmedizinischen Therapie, enthaltend eine therapeutisch wirksame Menge mindestens einer Verbindung gemäß den Anspruchen 1 bis 3.

7. Medikamente zur Verwendung in der cardiovasculären Therapie, enthaltend eine therapeutisch wirksame Menge mindestens einer der Verbindungen gemäß den Ansprüchen 1 bis 3.

8. Medikament zur Verwendung in der antiallergischen Therapie, enthaltend eine therapeutisch wirksame Menge mindestens einer der Verbindungen gemäß den Ansprüchen 1 bis 3.

9. Medikament zur Verwendung in der psychotropen Therapie, enthaltend eine therapeutisch wirksame Menge mindestens einer der Verbindungen gemäß den Ansprüchen 1 bis 3.

10. Medikament zur Verwendung in der hypolipidämischen Therapie, enthaltend eine therapeutisch wirksame Menge mindestens einer der Verbindungen gemäß den Ansprüchen 1 bis 3.

11. Pharmazeutische oder veterinärmedizinische Zusammensetzung, dadurch gekennzeichnet, daß sie als Wirkstoff mindestens eine Verbindung gemäß den Ansprüchen 1 bis 3 zusammen mit einem pharmazeutischen Träger oder einem geeigneten Exzipienten enthält.

**Claims**

1. Products having the general formula (I):

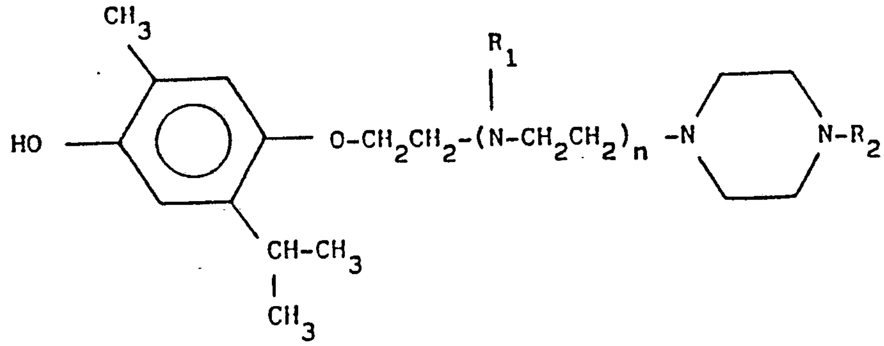

(I)

wherein

n is 0 or 1,

$R_1$ is a hydrogen atom or amethyl group, and

$R^2$ represents either a phenyl ring which is substituted or not by one more identical or different substituents selected from the group consisting of hydroxy, $C_1$-$C_3$ alkoxy (for example methoxy, ethoxy, propoxy), trifluouromethyl, chloro, fluoro or $C_1$-$C_2$ alkyl (for exemple methyl or ethyl) radicals, or 2-, 3- or 4-pyridyl ring, as a free base or as pharmaceutically compativle addition salts.

2. Products according to claim 1, characterized in that n = 0.

3. Products according to claim 1, characterized in that n = 1.

4. Process for preparing products according to claim 1, charactcrized in that a 4-(2-halogenoethoxy)-5-isopropyl-2-methyl phenol and an arylpiperazine having the general formula (IIa) in which

n is equal to 0 or 1,

$R_1$ represents a hydrogen atom or a methyl group and

$R_2$ represents either a phenyl ring which is substituted or not by one or more identical or different substituents selected from the group consisting of hydroxy, $C_1$-$C_3$ alkoxy (for example methoxy, ethoxy, propoxy), trifluoromethyl, chloro, fluoro or $C_1$-$C_2$ alkyl (for example methyl or ethyl) radicals, or a 2-, 3- or 4-pyridyl ring, the term halogeno representing a chlorine or bromine atom, are reacted together.

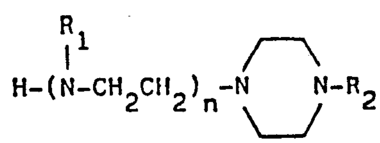

(IIa)

5. Process for preparing products according to claim 3, characterized in that a 1-halogenoethyl-4-arylpiperazine of the general formula (III):

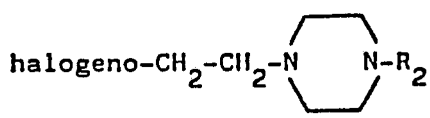

(III)

wherein

the term halogeno represents a bromine or chlorine atom and

$R_2$ represents either a phenyl ring which is substituted or not by one or more identical or different substituents selected from the group consisting of hydroxy, $C_1$-$C_3$ alkoxy (for example methoxy, ethoxy, propoxy), trifluoromethyl, chloro, fluoro or $C_1$-$C_2$ alkyl (for example methyl or ethyl) radicals, or a 2-, 3- or 4-pyridyl ring, is reacted with a 5-isopropyl-2-methyl-4-aminoethoxy phenol of the general formula (IV)

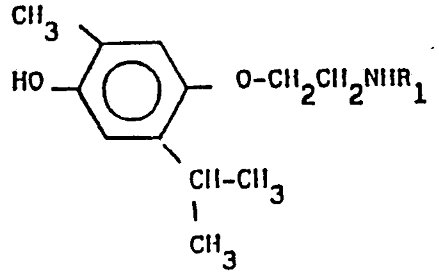

(IV)

in which $R_1$ = H or methyl.

6. Pharmaceutical products which are useful in human or veterinary therapeutics, containing a

therapeutically efficient amount of at least one compound according to claims 1 to 3.

7. Pharmaceutical products which are useful in cardiovascular therapeutics, containing a therapeutically efficient amount of at least one compound according to claims 1 to 3.

8. Pharmaceutical products which are useful in antiallergic therapeutics, containing a therapeutically efficient amount of at least one compound according to claims 1 to 3.

9. Pharmaceutical products which are useful in psychotropic therapeutics, containing a therapeutically efficient amount of at least one compound according to claims 1 to 3.

10. Pharmaceutical products which are useful in hypolipidemient therapeutics, containing a therapeutically efficient amount of at least one compound according to claims 1 to 3.

11. Pharmaceutical or veterinary composition, characterized in that it comprises, as an active principle, at least one product according to claims 1 to 3 in association with a suitable pharmaceutical vehicle or excipient.